# EUROPEAN PATENT APPLICATION

(11) **EP 4 421 189 A1**
(43) Date of publication of application: **28.08.2024**
(21) Application number: 23382178.4
(22) Date of filing: 27.02.2023
(51) Int. Cl.: C12Q 1/6883

(54) **METHOD FOR THE DIAGNOSIS OR PROGNOSIS OF HUNTINGTON'S DISEASE**

(71) Applicant: Universitat de Barcelona, 08028 Barcelona (ES); Consorcio Centro de Investigación Biomédica en Red, 28029 Madrid (ES); Fundació Institut de recerca de l'Hospital de la Santa Creu i Sant Pau, 08041 Barcelona (ES)
(72) Inventor: MARTI PUIG, Eulalia, 08028 Barcelona (ES); GÁMEZ VALERO, Ana, 08028 Barcelona (ES); HERRERO LORENZO, Marina, 08028 Barcelona (ES); ESCARAMIS BABIANO, Georgia, 28029 Madrid (ES); KULISEVSKY BOJARSKI, Jaime, 08041 Barcelona (ES); PÉREZ PÉREZ, Jesús, 08041 Barcelona (ES); MARTÍNEZ HORTA, Saül, Indra, 08041 Barcelona (ES)
(74) Representative: Hoffmann Eitle

(57) **Abstract**

The present invention refers to an *in vitro* method for the diagnosis or prognosis of Huntington's disease, preferably for the early diagnosis or prognosis of Huntington's disease. Said method involves assessing the level of expression of a tRNA fragment that comprises the 5'end of tRNA^{Glu/CTC} (tRF-5^{Glu/CTC} ) or 5'end of tRNA^{Gly/GCC} (tRF-5^{Gly/GCC} ) in a biological sample obtained from the subject, wherein the identification of a deviation in the expression level with respect to a pre-established threshold value determined in healthy subjects is an indication that the subject may be suffering from Huntington's disease or has a poor prognosis.

## Description

### FIELD OF THE INVENTION

The present invention refers to the medical field. Particularly, the present invention refers to an *in vitro* method for the diagnosis or prognosis of Huntington's disease (HD), preferably for the early diagnosis or prognosis of HD.

### STATE OF THE ART

HD is a dominantly inherited neurodegenerative disorder caused by a CAG triplet repeat expansion in the Huntingtin gene (HTT). Neuropathologically, HD is characterized by the deposition of the mutant HTT protein in the brain and the progressive loss of specific neurons in the striatum and the cortex. Although the diagnosis of HD is preferentially determined by family history and genetic testing, clinical diagnosis and age at onset are predominantly defined by the presence of unequivocal motor abnormalities such as chorea, dystonia, parkinsonism and gait abnormalities. Generally, this has been designated as the HD manifest stage (M-HD). Nevertheless, diverse disease landmarks such as progressive brain changes, and distinct cognitive, behavioural and emotional disturbances precede the onset and define the traditionally known as the premanifest stage (P-HD).

Current HD treatments provide only symptomatic relief and later results on disease-modifying therapeutic clinical trials have shown no efficacy, which has raised the concern of the scientific community. Most of these clinical trials enrolled predominately patients in the manifest phase of the disease, as it appears more feasible to demonstrate and measure a clinical benefit. The lack of sensitive molecular indicators of pathobiological processes has limited the inclusion of P-HD patients in these trials. Hence, the combination of predictive genetic testing with new early molecular biomarkers could provide an opportunity to improve clinical decision-making and, eventually, to delay the appearance of clinical symptoms.

Later efforts on neurodegenerative diseases, including HD, have been invested in the definition of biofluid-based minimally invasive biomarkers. Specifically, it has been shown that plasma can reflect molecular changes occurring in these heterogeneous disorders. In HD, the most remarkable biofluid biomarker is mutant HTT, which is utilized as a measurement of HTT reduction in genetic interventional studies. Similarly, plasma neurofilament light chain (NfL) shows a high prognostic value for HD but it is not disease-specific, as it is also observed in other neurodegenerative and brain-related diseases. Otherwise, regarding RNA, miRNAs have been largely studied as interesting transcriptomic biomarkers in peripheral circulation due to their stability outside cells. Moreover, we recently showed that small RNAs (sRNAs, < 200 nucleotides) may have a profound role in HD pathogenesis. The intrastriatal injection of sRNAs from HD patients' brains triggers motor abnormalities, neuronal toxicity, and transcriptional alteration in naive mice. These results suggest that different types of sRNAs can be responsible for transcriptomic deregulation occurring during the disease. Therefore, the understanding of sRNA expression dynamics should provide clues about the progression of the disease and help to elucidate if sRNA perturbations are an early phenomenon in HD.

In peripheral circulation, extracellular sRNAs (exRNAs) can be found as freely circulating, associated to lipid- or protein-complexes, or encapsulated inside different types of vesicles. Specifically, extracellular vesicles (EVs) are bilayered vesicles produced by the majority of cells with the capacity to package and deliver molecules such as RNAs and proteins between cells, making them attractive in biomarker discovery.

There is an unmet medical need of finding reliable strategies for the diagnosis or prognosis of HD. The present invention is focused on solving this problem and an innovative method aimed at the diagnosis or prognosis of HD is herein in provided.

### DESCRIPTION OF THE INVENTION

As explained above, the present invention refers to an *in vitro* method for the diagnosis or prognosis of HD, preferably for the early diagnosis or prognosis of this disease.

The inventors of the present invention have defined premanifest HD-specific biomarkers sensitive enough to stratify patients, and/or to monitor changes prior to clinical decision.

In a first step, the plasma exRNA transcriptome was explored, in healthy volunteers' samples, using an exhaustive pipeline of analysis. Then, the same strategy was conducted focusing on two well-differentiated plasma subfractions, EVs-enriched and Non-EV pools, from HD patients in comparison to control samples. This design may highlight poorly represented exRNA species that can be masked when studying sRNA from total blood/plasma samples. Importantly, the inventors of the present invention observed that most exRNAs in plasma EVs are downregulated in HD and they validated these results in biological and technical replicates by an additional technique. Lastly, the inventors of the present invention showed that early deregulation of specific exRNAs in HD correlates with premanifest cognitive alterations and they tested that specific exRNAs (individually and combined) show a great diagnostic accuracy at premanifest stages, similar to plasma NfL, which suppose the definition of novel prognostic biomarkers.

Particularly, as a proof of concept, the RNAs shown in **Table 1** were analysed in the present invention, both individually and combinations thereof:

**Table 1**

| **RNA** | **SEQUENCE** | **SEQ ID** |
|---|---|---|
| tRNA^{Glu/CTC} | UCCCUGGUGGUCUAGUGGUUAGGAUUCGGCG | SEQ ID NO: 1 |
| tRNA^{Lys/TTT} | UAGCUCAGUUGGUAGAGC | SEQ ID NO: 2 |
| miR-451a | AAACCGUUACCAUUACUGAGUU | SEQ ID NO: 3 |
| miR-21-5p | UAGCUUAUCAGACUGAUGUUGA | SEQ ID NO: 4 |
| tRNA^{Gly/GCC} | GCAUUGGUGGUUCAGUGGUAGAAUUCUCGC | SEQ ID NO: 5 |
| let-7a-5p | UGAGGUAGUAGGUUGUAUAGUU | SEQ ID NO: 6 |
| miR-26a-5p | UUCAAGUAAUCCAGGAUAGGCU | SEQ ID NO: 7 |
| miR-27a-3p | UUCACAGUGGCUAAGUUCCGC | SEQ ID NO: 8 |

The statistical results of the individual biomarkers are shown in **Table 2** (P-HD vs control).

**Table 2**

| **RNA** | **AUC** | **p-value** |
|---|---|---|
| tRNA^{Glu/CTC} | 0.79 | 0.002 |
| tRNA^{Lys/TTT} | 0.625 | 0.249 |
| miR-451a | 0.703 | 0.031 |
| miR-21-5p | 0.745 | 0.008 |
| tRNA^{Gly/GCC} | 0.73 | 0.013 |
| let-7a-5p | 0.728 | 0.014 |
| miR-26a-5p | 0.663 | 0.092 |
| miR-27a-3p | 0.773 | 0.003 |

The statistical results of combinations of two biomarkers are shown in **Table 3** (P-HD vs control).

**Table 3**

| **RNA** | **AUC** | **p-value** |
|---|---|---|
| tRNA^{Glu/CTC} | 0.793 | 0.001 |
| tRNA^{Lys/TTT} | | |
| miR-451a | 0.773 | 0.001 |
| miR-21-5p | | |
| **tRNA^{Gly/GCC}** | **0.82** | **0.0001** |
| **let-7a** | | |
| miR-26a | 0.783 | 0.002 |
| miR-27a | | |
| **tRNA^{Glu/CTC}** | **0.82** | **0.0001** |
| **miR-21-5p** | | |
| miR-451a | 0.775 | 0.003 |
| tRNA^{Lys/TTT} | | |
| **tRNA^{Gly/GCC}** | **0.863** | **0.0001** |
| **miR-27a** | | |
| miR-26a | 0.753 | 0.006 |
| let-7a | | |
| **tRNA^{Glu/CTC}** | **0.81** | **0.001** |
| **miR-451a** | | |
| miR-21-5p | 0.75 | 0.007 |
| tRNA^{Lys/TTT} | | |
| tRNA^{Gly/GCC} | 0.793 | 0.002 |
| miR-26a | | |
| **miR-27a** | **0.83** | **0.0001** |
| **let-7a** | | |
| tRNA^{Glu/CTC} | 0.805 | 0.001 |
| miR-26a | | |
| **miR-21-5p** | **0.778** | **0.003** |
| **let-7a** | | |
| tRNA^{Gly/GCC} | 0.765 | 0.004 |
| miR-451a | | |
| miR-27a | 0.788 | 0.002 |
| tRNA^{Lys/TTT} | | |
| **tRNA^{Glu/CTC}** | **0.825** | **0.0001** |
| **tRNA^{Gly/GCC}** | | |
| miR-451a | 0.715 | 0.02 |
| miR-26a | | |
| tRNA^{Lys/TTT} | 0.795 | 0.001 |
| let-7a | | |
| miR-21-5p | 0.79 | 0.002 |
| miR-27a | | |
| **tRNA^{Glu/CTC}** | **0.838** | **0.0001** |
| **miR-27a** | | |
| miR-451a | 0.78 | 0.002 |
| let-7a | | |
| tRNA^{Lys/TTT} | 0.668 | 0.07 |
| miR-26a | | |
| tRNA^{Gly/GCC} | 0.83 | 0.0001 |
| miR-21-5p | | |
| **tRNA^{Glu/CTC}** | **0.833** | **0.0001** |
| **let-7a** | | |
| tRNA^{Gly/GCC} | 0.738 | 0.01 |
| tRNA^{Lys/TTT} | | |
| miR-27a | 0.798 | 0.001 |
| miR-451a | | |
| miR-26a | 0.748 | 0.007 |
| miR-21-5p | | |

The statistical results of combinations of three biomarkers are shown in **Table 4** (P-HD vs control).

**Table 4**

| **RNA** | **AUC** | **p-value** |
|---|---|---|
| tRNA^{Gly/GCC} | 0.848 | 0.0001 |
| let-7a | | |
| tRNA^{Glu/CTC} | | |
| tRNA^{Gly/GCC} | 0.853 | 0.0001 |
| let-7a | | |
| miR-21-5p | | |
| **tRNA^{Gly/GCC}** | **0.860** | **0.0001** |
| **let-7a** | | |
| **miR-27a** | | |
| tRNA^{Gly/GCC} | 0.828 | 0.0001 |
| let-7a | | |
| miR-451a | | |
| **tRNA^{Glu/CTC}** | **0.860** | **0.0001** |
| **miR-21-5p** | | |
| tRNA^{Gly/GCC} | | |
| tRNA^{Glu/CTC} | 0.843 | 0.0001 |
| miR-21-5p | | |
| let-7a | | |
| tRNA^{Glu/CTC} | 0.835 | 0.0001 |
| miR-21-5p | | |
| miR-27a | | |
| tRNA^{Glu/CTC} | 0.833 | 0.0001 |
| miR-21-5p | | |
| miR-451a | | |
| **miR-27a** | **0.86** | **0.0001** |
| **let-7a** | | |
| **tRNA^{Glu/CTC}** | | |
| miR-27a | 0.825 | 0.0001 |
| let-7a | | |
| miR-21-5p | | |
| miR-27a | 0.83 | 0.0001 |
| let-7a | | |
| miR-451a | | |
| tRNA^{Glu/CTC} | 0.843 | 0.0001 |
| miR-451a | | |
| miR-27a | | |
| tRNA^{Glu/CTC} | 0.85 | 0.0001 |
| miR-451a | | |
| let-7a | | |
| tRNA^{Glu/CTC} | 0.84 | 0.0001 |
| miR-451a | | |
| tRNA^{Gly/GCC} | | |
| miR-21-5p | 0.79 | 0.002 |
| let-7a | | |
| miR-451a | | |
| miR-21-5p | 0.803 | 0.001 |
| miR-27a | | |
| miR-451a | | |
| **tRNA^{Glu/CTC}** | **0.875** | **0.0001** |
| **tRNA^{Gly/GCC}** | | |
| **miR-27a** | | |
| **tRNA^{Gly/GCC}** | **0.86** | **0.0001** |
| **miR-27a** | | |
| **miR-451a** | | |

The statistical results of combinations of four biomarkers are shown in **Table 5** (P-HD vs control).

**Table 5**

| **RNA** | **AUC** | **p-value** |
|---|---|---|
| tRNA^{Gly/GCC} | 0.863 | 0.0001 |
| let-7a | | |
| miR-21-5p | | |
| miR-27a | | |
| tRNA^{Gly/GCC} | 0.858 | 0.0001 |
| let-7a | | |
| miR-27a | | |
| miR-451a | | |
| tRNA^{Glu/CTC} | 0.865 | 0.0001 |
| miR-21-5p | | |
| tRNA^{Gly/GCC} | | |
| miR-451a | | |
| **tRNA^{Glu/CTC}** | **0.878** | **0.0001** |
| **miR-21-5p** | | |
| **tRNA^{Gly/GCC}** | | |
| **miR-27a** | | |
| tRNA^{Glu/CTC} | 0.85 | 0.0001 |
| miR-21-5p | | |
| tRNA^{Gly/GCC} | | |
| let-7a | | |
| tRNA^{Glu/CTC} | 0.868 | 0.0001 |
| miR-451a | | |
| tRNA^{Gly/GCC} | | |
| miR-27a | | |
| tRNA^{Glu/CTC} | **0.873** | **0.0001** |
| **let-7a** | | |
| tRNA^{Gly/GCC} | | |
| **miR-27a** | | |
| tRNA^{Gly/GCC} | 0.858 | 0.0001 |
| miR-27a | | |
| miR-451a | | |
| let-7a | | |
| tRNA^{Gly/GCC} | 0.858 | 0.0001 |
| miR-27a | | |
| miR-451a | | |
| miR-21-5p | | |
| miR-27a | 0.85 | 0.0001 |
| let-7a | | |
| tRNA^{Glu/CTC} | | |
| miR-21-5p | | |
| miR-27a | 0.853 | 0.0001 |
| let-7a | | |
| tRNA^{Glu/CTC} | | |
| miR-451a | | |
| tRNA^{Glu/CTC} | 0.855 | 0.0001 |
| miR-21-5p | | |
| tRNA^{Gly/GCC} | | |
| miR-26a-5p | | |
| tRNA^{Glu/CTC} | 0.86 | 0.0001 |
| miR-21-5p | | |
| tRNA^{Gly/GCC} | | |
| tRNA^{Lys/TTT} | | |

The statistical results of combinations of five biomarkers are shown in **Table 6** (P-HD vs control).

**Table 6**

| | **AUC** | **p-value** |
|---|---|---|
| tRNA^{Glu/CTC} | 0.868 | 0.0001 |
| miR-21-5p | | |
| tRNA^{Gly/GCC} | | |
| miR-27a | | |
| miR-451a | | |

The statistical results of the combination of the biomarkers of the invention with NfL are shown in the tables below. In this regard, please note that for the NfL (gold standard) an AUC = 0.815 and p-value of 0.002 were obtained.

Particularly, the statistical results of the combination of the biomarkers of the invention with NfL are shown in **Table 7** (P-HD vs control).

**Table 7**

| | **AUC** | **p-value** |
|---|---|---|
| tRNA^{Glu/CTC} | **0.88** | **0.001** |
| **NfL** | | |
| tRNA^{Gly/GCC} | 0.861 | 0.001 |
| NfL | | |
| **miR-451a** | **0.89** | **0.0001** |
| **NfL** | | |
| **NfL** | **0.895** | **0.0001** |
| **miR-27a** | | |

The statistical results of the combination of two biomarkers of the invention with NfL are shown in **Table 8** (P-HD vs control).

**Table 8**

| | **AUC** | **p-value** |
|---|---|---|
| tRNA^{Glu/CTC} | **0.919** | **0.0001** |
| **miR-21-5p** | | |
| **NfL** | | |
| tRNA^{Glu/CTC} | 0.89 | 0.001 |
| miR-26a | | |
| NfL | | |
| tRNA^{Glu/CTC} | **0.923** | **0.0001** |
| **let-7a** | | |
| **NfL** | | |
| tRNA^{Glu/CTC} | 0.909 | 0.0001 |
| miR-27a | | |
| NfL | | |
| tRNA^{Glu/CTC} | 0.904 | 0.0001 |
| miR-451a | | |
| NfL | | |
| tRNA^{Glu/CTC} | 0.885 | 0.001 |
| tRNA^{Gly/GCC} | | |
| NfL | | |
| tRNA^{Gly/GCC} | 0.904 | 0.0001 |
| miR-21-5p | | |
| NfL | | |
| tRNA^{Gly/GCC} | 0.866 | 0.001 |
| miR-26a | | |
| NfL | | |
| tRNA^{Gly/GCC} | 0.914 | 0.0001 |
| let-7a | | |
| NfL | | |
| tRNA^{Gly/GCC} | 0.895 | 0.0001 |
| miR-27a | | |
| NfL | | |
| tRNA^{Gly/GCC} | 0.904 | 0.0001 |
| miR-451a | | |
| NfL | | |
| **miR-451a** | **0.928** | **0.0001** |
| **miR-27a** | | |
| **NfL** | | |

The statistical results of the combination of three biomarkers of the invention with NfL are shown in **Table 9** (P-HD vs control).

**Table 9**

| | **AUC** | **p-value** |
|---|---|---|
| tRNA^{Gly/GCC} | 0.904 | 0.0001 |
| miR-27a | | |
| tRNA^{Glu/CTC} | | |
| NfL | | |
| tRNA^{Glu/CTC} | 0.914 | 0.0001 |
| miR-27a | | |
| miR-451a | | |
| NfL | | |
| tRNA^{Gly/GCC} | 0.89 | 0.0001 |
| miR-26a | | |
| tRNA^{Glu/CTC} | | |
| NfL | | |
| **miR-21-5p** | **0.933** | **0.0001** |
| **miR-27a** | | |
| **miR-451a** | | |
| **NfL** | | |
| tRNA^{Gly/GCC} | 0.885 | 0.001 |
| tRNA^{Lys/TTT} | | |
| tRNA^{Glu/CTC} | | |
| NfL | | |
| tRNA^{Gly/GCC} | **0.928** | **0.0001** |
| **miR-27a** | | |
| **miR-451a** | | |
| **NfL** | | |
| tRNA^{Glu/CTC} | 0.919 | 0.0001 |
| miR-27a | | |
| miR-21-5p | | |
| NfL | | |

The statistical results of the combination of four biomarkers of the invention with NfL are shown in **Table 10** (P-HD vs control).

**Table 10**

| | **AUC** | **p-value** |
|---|---|---|
| tRNA^{Gly/GCC} | 0.914 | 0.0001 |
| miR-451a | | |
| tRNA^{Glu/CTC} | | |
| NfL | | |
| miR-27a | | |
| tRNA^{Gly/GCC} | 0.923 | 0.0001 |
| miR-21-5p | | |
| tRNA^{Glu/CTC} | | |
| NfL | | |
| miR-27a | | |
| **let-7** | **0.928** | **0.0001** |
| **miR-451a** | | |
| tRNA^{Glu/CTC} | | |
| **NfL** | | |
| **miR-27a** | | |

The statistical results of the combination of the eight biomarkers of the invention (see **Table 1**) with NfL are shown in **Table 11** (P-HD vs control). Initially, the combination of the eight biomarkers of **Table 1** with NfL is shown in the first row of **Table 11.** Moreover, the same assay was carried out but eliminating some of the eight biomarkers: miR-26a-5p (-miR-26a-5p), miR-26a-5p and tRNA^{Gly/GCC} (-miR-26a-5p and tRNA^{Gly/GCC}), miR-26a-5p and miR451a (-miR-26a-5p and miR451a) and miR-26a-5p and tRNA^{Lys/TTT} (-miR-26a-5p and tRNA^{Lys/TTT}). The results are shown in the second row of **Table 11.**

**Table 11**

| | **AUC** | **p-value** |
|---|---|---|
| **8 biomarkers** + **NfL** | **0.943** | **0.0001** |
| (- **miR-26a-5p)** + **NfL** | **0.943** | **0.0001** |
| (- miR-26a-5p and tRNA^{Gly/GCC}) + NfL | 0.933 | 0.0001 |
| (- miR-26a-5p and miR451a) + NfL | 0.919 | 0.0001 |
| (- miR-26a-5p and tRNA^{Lys/TTT}) + NfL | 0.938 | 0.0001 |

So, as shown in **Tables 1** to **11** above, although different RNA biomarkers are associated with HD, tRNA^{Glu/CTC} and tRNA^{Gly/GCC} show relevant results both individually and combined with other biomarkers. The best results obtained with tRNA^{Glu/CTC} and tRNA^{Gly/GCC}, and combinations with other biomarkers, are summarized in **Table 12.**

**Table 12**

| **Biomarker** | **AUC** | **p-value** |
|---|---|---|
| tRNA^{Gly/GCC} + tRNA^{Glu/CTC} | **0.83** | 0.0001 |
| tRNA^{Gly/GCC} + miR-27 | **0.86** | 0.0001 |
| tRNA^{Gly/GCC} + tRNA^{Glu/CTC} + miR-27 | **0.88** | 0.0001 |
| tRNA^{Gly/GCC} + tRNA^{Glu/CTC}+ miR-27 + miR-21 | **0.88** | 0.0001 |
| tRNA^{Glu/CTC} + NfL | **0.86** | 0.0001 |
| tRNA^{Glu/CTC}+ miR-21 + NfL | **0.896** | 0.0001 |
| tRNA^{Gly/GCC} + miR-27 + miR451a + NfL | **0.904** | 0.0001 |
| tRNA^{Glu/CTC} + tRNA^{Lys/TTT} + miR-451a + miR-21-5p + tRNA^{Gly/GCC} + let-7a-5p + miR-27a-3p + NfL | **0.93** | 0.0001 |
| tRNA^{Glu/CTC} + miR-451a + miR-21-5p + tRNA^{Gly/GCC} + let-7a-5p + miR-27a-3p + NfL | **0.922** | 0.0001 |

On the other hand, in order to interpret the invention, it is highly important to consider that tRNA^{Glu/CTC} (SEQ ID NO: 1) and tRNA^{Gly/GCC} (SEQ ID NO: 5) were assayed, as proof of concept, in the present invention as. However, since tRNA^{Glu/CTC} (SEQ ID NO: 1) and tRNA^{Gly/GCC} (SEQ ID NO: 5) pertain to a very specific group of tRNAs because they are tRNA fragments which comprise the 5'end of tRNA^{Glu/CTC} (tRF-5^{Glu/CTC}) or 5'end of tRNA^{Gly/GCC} (tRF-5^{Gly/GCC}), it is plausible to think that any tRNA fragment which comprises the 5'end of tRNA^{Glu/CTC} (tRF-5^{Glu/CTC}) or 5'end of tRNA^{Gly/GCC} (tRF-5^{Gly/GCC}) might be used for the diagnosis or prognosis of HD. In fact, **Table 13** and **Table 14** show other sequences which comprise the 5'end of tRNA^{Glu/CTC} (tRF-5^{Glu/CTC}) or 5'end of tRNA^{Gly/GCC}.

Please note that SEQ ID NO: 1 is underlined in **Table 13** to show that sequences SEQ ID NO: 9 to 41 comprise the SEQ ID NO: 1; and that SEQ ID NO: 5 is underlined in **Table 14** to show that sequences SEQ ID NO: 42 to 75 comprise the SEQ ID NO: 5.

**Table 13**

| **tRF-5^{Glu/CTC}** | |
|---|---|
| **SEQUENCE** | **SEQ ID** |
| UCCCUGGUGGUCUAGUGGUUAGGAUUCGGCG | SEQ ID NO: 1 |
| UCCCUGGUGGUCUAGUGGUUAGGAUUCGGCGC | SEQ ID NO: 9 |
| UCCCUGGUGGUCUAGUGGUUAGGAUUCGGCGCU | SEQ ID NO: 10 |
| UCCCUGGUGGUCUAGUGGUUAGGAUUCGGCGCUC | SEQ ID NO: 11 |
| UCCCUGGUGGUCUAGUGGUUAGGAUUCGGCGCUCU | SEQ ID NO: 12 |
| UCCCUGGUGGUCUAGUGGUUAGGAUUCGGCGCUCUC | SEQ ID NO: 13 |
| UCCCUGGUGGUCUAGUGGUUAGGAUUCGGCGCUCUCA | SEQ ID NO: 14 |
| UCCCUGGUGGUCUAGUGGUUAGGAUUCGGCGCUCUCAC | SEQ ID NO: 15 |
| UCCCUGGUGGUCUAGUGGUUAGGAUUCGGCGCUCUCACC | SEQ ID NO: 16 |
| UCCCUGGUGGUCUAGUGGUUAGGAUUCGGCGCUCUCACCG | SEQ ID NO: 17 |
| UCCCUGGUGGUCUAGUGGUUAGGAUUCGGCGCUCUCACCGC | SEQ ID NO: 18 |
| UCCCUGGUGGUCUAGUGGUUAGGAUUCGGCGCUCUCACCGCC | SEQ ID NO: 19 |
| UCCCUGGUGGUCUAGUGGUUAGGAUUCGGCGCUCUCACCGCCG | SEQ ID NO: 20 |
| UCCCUGGUGGUCUAGUGGUUAGGAUUCGGCGCUCUCACCGCCGC | SEQ ID NO: 21 |
| UCCCUGGUGGUCUAGUGGUUAGGAUUCGGCGCUCUCACCGCCGCGG | SEQ ID NO: 22 |
| UCCCUGGUGGUCUAGUGGUUAGGAUUCGGCGCUCUCACCGCCGCGGC | SEQ ID NO: 23 |
| UCCCUGGUGGUCUAGUGGUUAGGAUUCGGCGCUCUCACCGCCGCGGCCCG | SEQ ID NO: 24 |
| AUCCCUGGUGGUCUAGUGGUUAGGAUUCGGCG | SEQ ID NO: 25 |
| AUCCCUGGUGGUCUAGUGGUUAGGAUUCGGCGC | SEQ ID NO: 26 |
| AUCCCUGGUGGUCUAGUGGUUAGGAUUCGGCGCU | SEQ ID NO: 27 |
| AUCCCUGGUGGUCUAGUGGUUAGGAUUCGGCGCUCU | SEQ ID NO: 28 |
| CUCCCUGGUGGUCUAGUGGUUAGGAUUCGGCG | SEQ ID NO: 29 |
| CUCCCUGGUGGUCUAGUGGUUAGGAUUCGGCGC | SEQ ID NO: 30 |
| CUCCCUGGUGGUCUAGUGGUUAGGAUUCGGCGCU | SEQ ID NO: 31 |
| CUCCCUGGUGGUCUAGUGGUUAGGAUUCGGCGCUC | SEQ ID NO: 32 |
| CUCCCUGGUGGUCUAGUGGUUAGGAUUCGGCGCUCU | SEQ ID NO: 33 |
| CUCCCUGGUGGUCUAGUGGUUAGGAUUCGGCGCUCUC | SEQ ID NO: 34 |
| CUCCCUGGUGGUCUAGUGGUUAGGAUUCGGCGCUCUCACC | SEQ ID NO: 35 |
| GUCCCUGGUGGUCUAGUGGUUAGGAUUCGGCGCU | SEQ ID NO: 36 |
| UUCCCUGGUGGUCUAGUGGUUAGGAUUCGGCG | SEQ ID NO: 37 |
| UUCCCUGGUGGUCUAGUGGUUAGGAUUCGGCGC | SEQ ID NO: 38 |
| UUCCCUGGUGGUCUAGUGGUUAGGAUUCGGCGCU | SEQ ID NO: 39 |
| UUCCCUGGUGGUCUAGUGGUUAGGAUUCGGCGCUC | SEQ ID NO: 40 |
| UUCCCUGGUGGUCUAGUGGUUAGGAUUCGGCGCUCU | SEQ ID NO: 41 |

**Table 14**

| **tRF-5^{Gly/GCC}** | |
|---|---|
| **SEQUENCE** | **SEQ ID** |
| GCAUUGGUGGUUCAGUGGUAGAAUUCUCGC | SEQ ID NO: 5 |
| GCAUUGGUGGUUCAGUGGUAGAAUUCUCGCC | SEQ ID NO: 42 |
| GCAUUGGUGGUUCAGUGGUAGAAUUCUCGCCU | SEQ ID NO: 43 |
| GCAUUGGUGGUUCAGUGGUAGAAUUCUCGCCUG | SEQ ID NO: 44 |
| GCAUUGGUGGUUCAGUGGUAGAAUUCUCGCCUGCCACGCGGGAGGCCCGG | SEQ ID NO: 45 |
| GCAUUGGUGGUUCAGUGGUAGAAUUCUCGCCUGCCACG | SEQ ID NO: 46 |
| GCAUUGGUGGUUCAGUGGUAGAAUUCUCGCCUGCC | SEQ ID NO: 47 |
| GCAUUGGUGGUUCAGUGGUAGAAUUCUCGCCUGC | SEQ ID NO: 48 |
| GCAUUGGUGGUUCAGUGGUAGAAUUCUCGCCUGCCA | SEQ ID NO: 49 |
| GCAUUGGUGGUUCAGUGGUAGAAUUCUCGCCUGCCAC | SEQ ID NO: 50 |
| GCAUUGGUGGUUCAGUGGUAGAAUUCUCGCCUGCCACGC | SEQ ID NO: 51 |
| GCAUUGGUGGUUCAGUGGUAGAAUUCUCGCCUGCCACGCGGGA | SEQ ID NO: 52 |
| CGCAUUGGUGGUUCAGUGGUAGAAUUCUCGCCU | SEQ ID NO: 53 |
| CGCAUUGGUGGUUCAGUGGUAGAAUUCUCGCCUG | SEQ ID NO: 54 |
| CGCAUUGGUGGUUCAGUGGUAGAAUUCUCGCCUGC | SEQ ID NO: 55 |
| CGCAUUGGUGGUUCAGUGGUAGAAUUCUCGCC | SEQ ID NO: 56 |
| CGCAUUGGUGGUUCAGUGGUAGAAUUCUCGC | SEQ ID NO: 57 |
| CGCAUUGGUGGUUCAGUGGUAGAAUUCUCGCCUGCC | SEQ ID NO: 58 |
| GCAUUGGUGGUUCAGUGGUAGAAUUCUCGCCUGCCACGCGGGAG | SEQ ID NO: 59 |
| GCAUUGGUGGUUCAGUGGUAGAAUUCUCGCCUGCCACGCGGGAGGCC | SEQ ID NO: 60 |
| GCAUUGGUGGUUCAGUGGUAGAAUUCUCGCCUGCCACGCGGG | SEQ ID NO: 61 |
| GCAUUGGUGGUUCAGUGGUAGAAUUCUCGCCUGCCACGCGGGAGGC | SEQ ID NO: 62 |
| GCAUUGGUGGUUCAGUGGUAGAAUUCUCGCCUGCCACGCGG | SEQ ID NO: 63 |
| GCAUUGGUGGUUCAGUGGUAGAAUUCUCGCCUGCCACGCG | SEQ ID NO: 64 |
| GGCAUUGGUGGUUCAGUGGUAGAAUUCUCGCCU | SEQ ID NO: 65 |
| AGCAUUGGUGGUUCAGUGGUAGAAUUCUCGCCU | SEQ ID NO: 66 |
| UGCAUUGGUGGUUCAGUGGUAGAAUUCUCGCC | SEQ ID NO: 67 |
| AGCAUUGGUGGUUCAGUGGUAGAAUUCUCGCC | SEQ ID NO: 68 |
| GGCAUUGGUGGUUCAGUGGUAGAAUUCUCGC | SEQ ID NO: 69 |
| GGCAUUGGUGGUUCAGUGGUAGAAUUCUCGCC | SEQ ID NO: 70 |
| AGCAUUGGUGGUUCAGUGGUAGAAUUCUCGC | SEQ ID NO: 71 |
| GCAUUGGUGGUUCAGUGGUAGAAUUCUCGCCUGCCACGCGGGAGG | SEQ ID NO: 72 |
| GCAUUGGUGGUUCAGUGGUAGAAUUCUCGCCUGCCACGCGGGAGGCCC | SEQ ID NO: 73 |
| GCAUUGGUGGUUCAGUGGUAGAAUUCUCGCCUGCCACGCGGGAGGCCCG | SEQ ID NO: 74 |
| UGCAUUGGUGGUUCAGUGGUAGAAUUCUCGCCUG | SEQ ID NO: 75 |

| | |
|---|---|
| *Note: SEQ ID NO: 5 is underlined* | |

So, the first embodiment of the present invention refers to an *in vitro* method for the diagnosis or prognosis of HD (preferably for the early diagnosis or prognosis of HD) which comprises assessing the level of expression of a tRNA fragment which comprises the 5'end of tRNA^{Glu/CTC} (tRF-5^{Glu/CTC}) or 5'end of tRNA^{Gly/GCC} (tRF-5^{Gly/GCC}) in a biological sample obtained from the subject, wherein the identification of a deviation in the expression level (i.e., a higher or lower expression level) with respect to a pre-established threshold value determined in healthy subjects is an indication that the subject may be suffering from HD or has a poor prognosis (preferably that the subject may be suffering preclinical alterations associated to Huntington's disease or have a poor prognosis).

The second embodiment of the present invention refers to the *in vitro* use of a tRF-5^{Glu/CTC} or tRF-5^{Gly/GCC}, or of a kit comprising reagents for the detection of tRF-5^{Glu/CTC} or tRF-5^{Gly/GCC}, for the diagnosis or prognosis of HD.

In a preferred embodiment, the tRF-5^{Glu/CTC} sequence is selected from **Table 13.**

In a preferred embodiment, the present invention comprises assessing the level of expression of a tRF-5^{Glu/CTC} derived from the following tRNAs: tRNA^{Glu-CTC-1-1}, tRNA^{Glu-CTC-2-1}, tRNA^{Glu-CTC-1-5}, tRNA^{Glu-CTC-1-4}, tRNA^{Glu-CTC-1-3}, tRNA^{GluCTC-1-6}, tRNA^{Glu-CTC-1-2} or tRNA^{Glu-CTC-1-7}.

In a preferred embodiment, the tRF-5^{Gly/GCC} is selected from **Table 14.**

In a preferred embodiment, the present invention comprises assessing the level of expression of a tRF-5^{Gly/GCC} derived from the following tRNAs: tRNA5^{Gly-GCC-5-1}, tRNA5^{Gly-GCC-3-1}, tRNA5^{Gly-GCC-2-1}, tRNA5^{Gly-GCC-2-5}, tRNA5^{Gly-GCC-2-4}, tRNA5^{Gly-GCC-2-6}, tRNA5^{Gly-GCC-2-2} or tRNA5^{Gly-GCC-2-3}.

In a preferred embodiment, the present invention further comprises assessing the level of expression of miR-451a, miR-21-5p, miR-27a-3p, let-7a-5p, miR-26a-5p, a tRNA fragment comprising the 5'end of tRNA^{Lys/TTT} and/or Neurofilament light chain (NfL), or combinations thereof.

In a preferred embodiment, the present invention comprises assessing the level of expression of the combination of biomarkers of **Table 12.**

In a preferred embodiment, the present invention comprises a) measuring the expression level of the combinations of biomarkers, b) processing the expression level values in order to obtain a risk score, and c) wherein if the risk score obtained has a variation or deviation as compared with a reference value, this is indicative that the subject is suffering from HD or has a poor prognosis.

In a preferred embodiment, the biological sample obtained from the patient is total blood or blood fractions (including plasma, serum, and blood cells), cerebrospinal fluid, urine, saliva, or extracellular vesicles derived from any of these types of samples. The biological samples may also be tissue and cell samples (including brain homogenate, cultured fibroblasts, and iPSC derived, all of them from human or mouse models).

Alternatively, the present invention also refers to the following topics:
A method for detecting an RNA biomarker in a test sample from a human gene-mutation carrier of HD, the method comprising: a) contacting the test sample with a specific primer for the RNA biomarker, wherein the RNA biomarker comprises a tRNA fragment which comprises the 5'end of tRNA^{Glu/CTC} (tRF-5^{Glu/CTC}) or 5'end of tRNA^{Gly/GCC} (tRF-5^{Gly/GCC}); b) amplifying biomarker to produce an amplification product in the test sample; and c) measuring the expression level by determining the level of the amplification product in the test sample.

In a preferred embodiment, the present invention is a computer-implemented invention, wherein a processing unit (hardware) and a software are configured to: a) Receive the expression level values of any of the above cited biomarkers or signatures, b) process the expression level values received for finding substantial variations or deviations, and c) provide an output through a terminal display of the variation or deviation of the expression level, wherein the variation or deviation of the expression level indicates that the subject may be suffering from HD.

A computer program or a computer-readable media containing means for carrying out the computer-implemented invention.

For the purpose of the present invention the following terms are defined:
- 5'end tRNA, tRF-5, or 5-tRFs, according to the MINTbase tRFs database, are defined as a type of small RNA which are typically 18-50 nucleotides (nt) long and originate from the 5'-end of mature tRNAs. Specific S'tRFs may contain an additional nucleotide upstream of the 5'-end of the mature tRNAs. These 2 start positions are indicated as positions +1 or -1 respectively. Those S'tRFs that contain part of the codon loop are specifically named 5'-tRNA halves (identified as 5'-half).
- The expression "5' region", as used herein, refers to a region of a nucleotide strand that includes the 5' end of said strand. As used herein, the "5' region" and the "5' terminal region" refers to the same region of a nucleotide strand and may be used interchangeably. The term "5' end", as used herein, designates the end of a nucleotide strand that has the fifth carbon in the sugar-ring of the deoxyribose at its terminus.
- According to the present invention, a reference value can be a "pre-established threshold value" or a "cut-off' value. Typically, a "threshold value" or "cut-off value" can be determined experimentally, empirically, or theoretically. According to the present invention, the "pre-established threshold" value refers to a value previously determined in subjects who are nonsuffering from HD. Thus, for instance, the subject is likely to suffer from HD, or to have a poor prognosis, when there is a variation or deviation in the level of expression of the biomarkers of the invention (i.e., it may be a higher or a lower expression), as compared with a pre-established "threshold value". A "threshold value" can also be arbitrarily selected based upon the existing experimental and/or clinical conditions, as would be recognized by a person of ordinary skilled in the art. The "threshold value" has to be determined in order to obtain the optimal sensitivity and specificity according to the function of the test and the benefit/risk balance (clinical consequences of false positive and false negative). Typically, the optimal sensitivity and specificity (and so the "threshold value") can be determined using a Receiver Operating Characteristic (ROC) curve based on experimental data.
- The term "comprising" means including, but not limited to, whatever follows the word "comprising". Thus, use of the term "comprising" indicates that the listed elements are required or mandatory, but that other elements are optional and may or may not be present.
- The term "consisting of" means including, and limited to, whatever follows the phrase "consisting of". Thus, the phrase "consisting of" indicates that the listed elements are required or mandatory, and that no other elements may be present.
- The present invention also refers to- and includes "non-canonical sequence" which are slightly different sequences to the sequences of the present invention but biologically produced by imprecise and alternative cleavage of the precursor during biogenesis.

### Description of the figures

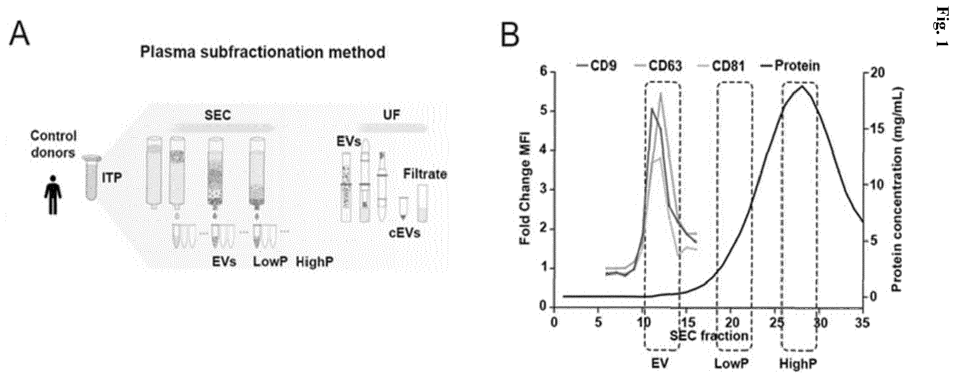

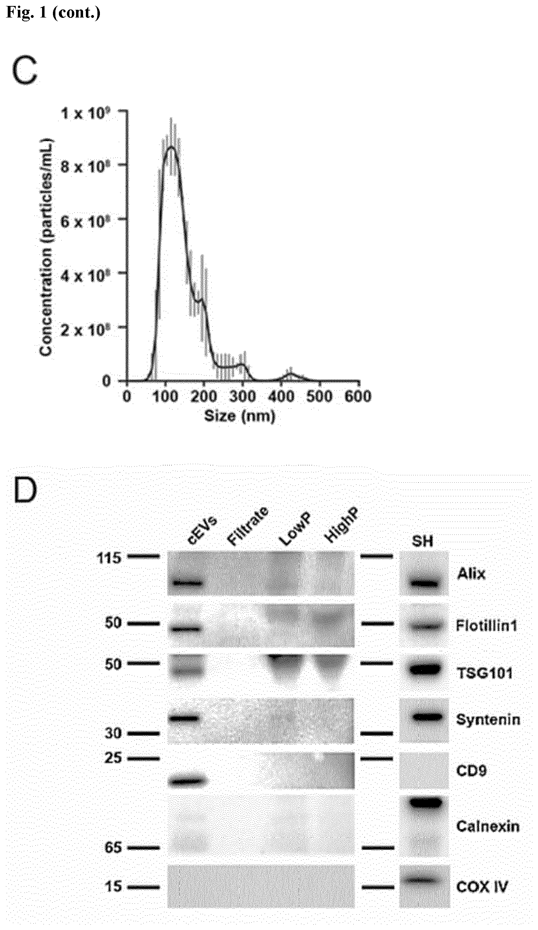

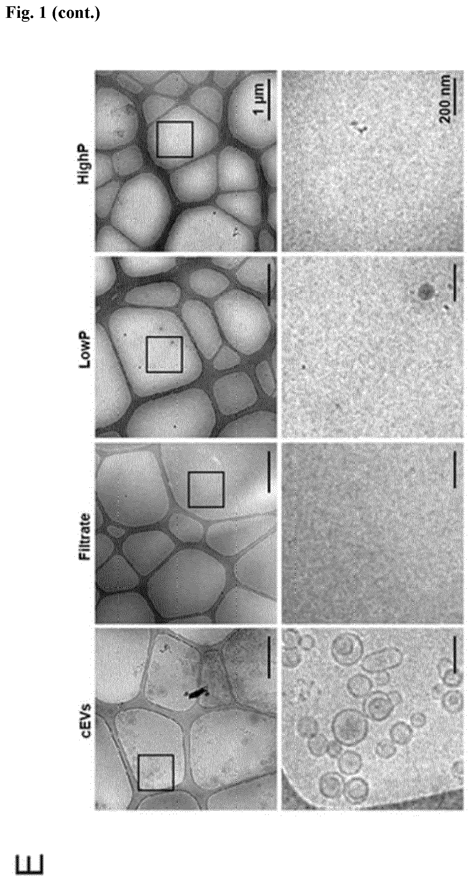

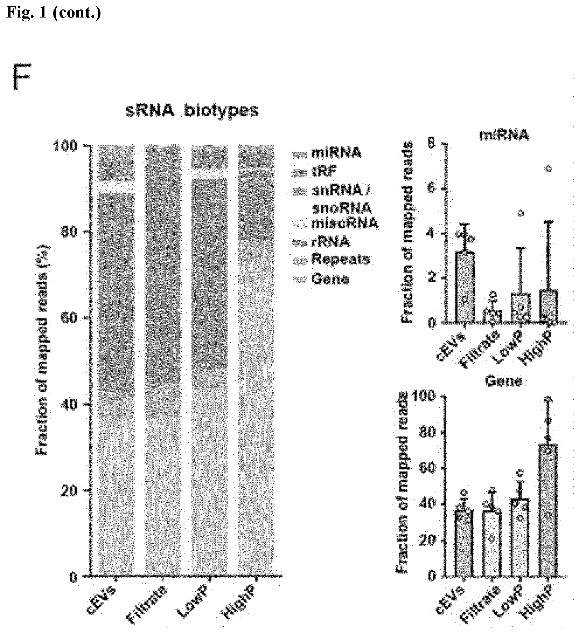

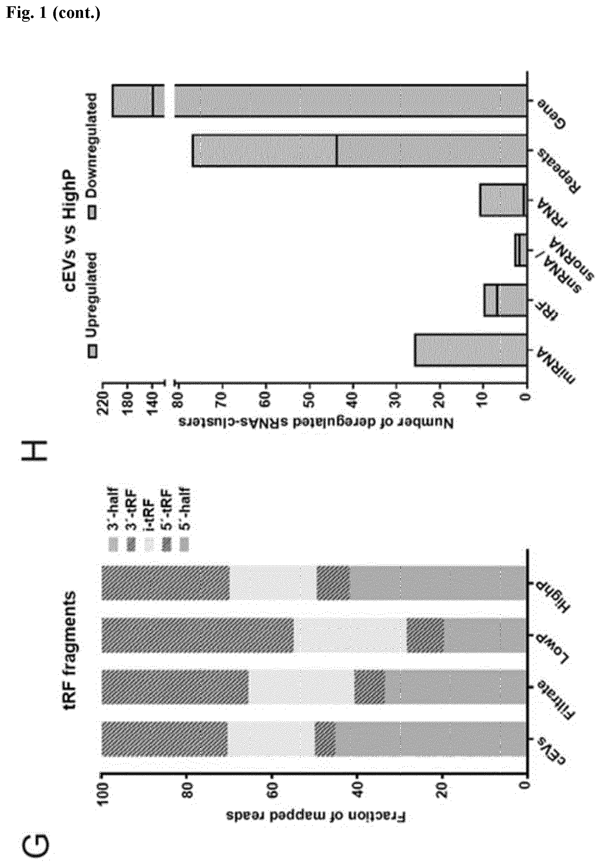

**Figure 1****. sRNA biotypes are differently distributed between plasma subfractions. (A)** Schematic representation of plasma-EV isolation procedure by SEC and UF and selected fractions for analysis: cEVs, Filtrate, Low-Protein (LowP) and High-Protein (HighP) fractions. **(B)** Representative SEC profile of plasma samples showing EV elution in low-protein fractions as measured by Bradford protein assay and enriched in CD9, CD63 and CD81 markers as analysed by flow cytometry. **(C)** Representative NTA size distribution profile of cEVs fraction (n=5). **(D)** Western Blot analysis of isolated fractions. Fractions were analysed for the presence of EV markers (Alix, Flotillin1, TSG101, Syntenin and CD9) and negative EV-markers (Calnexin and COX IV). SH-SY5Y cell lysate was used as positive controls (n=3). **(E)** Representative images of cryo-EM of the isolated fractions. Upper images, bar=1 µm. Lower images, bar=200nm. Using SeqCluster tool: **(F)** Fraction of reads that align to small RNA types are shown per plasma subfraction. **(G)** Classification of reads that align to tRF fragments in 3'-half, 3'-tRF, i-tRF, 5'-tRF, and 5'-half per plasma subfraction. **(H)** Total number of sRNAs-clusters differentially expressed between cEVs and HighP subfractions (n=5 per group, p-adj<0.05). Samples of each subfraction, n=5. Mean ± SD is shown.

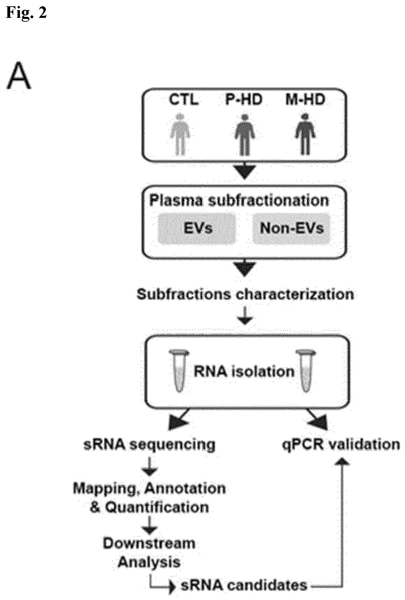

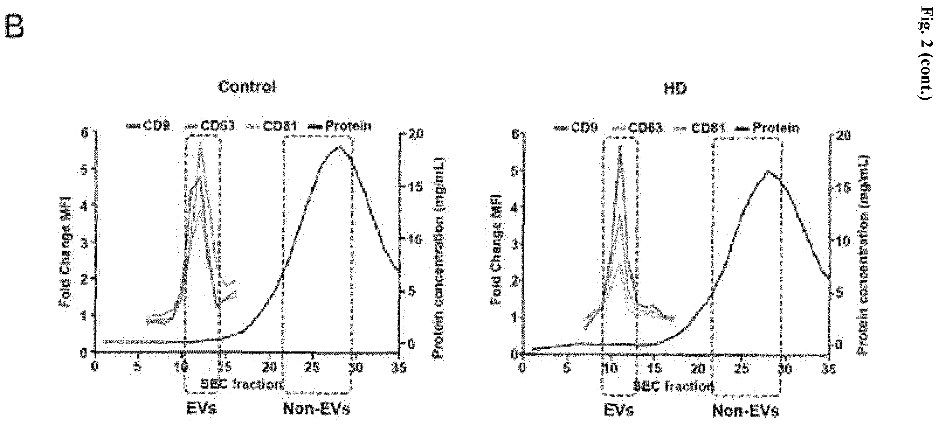

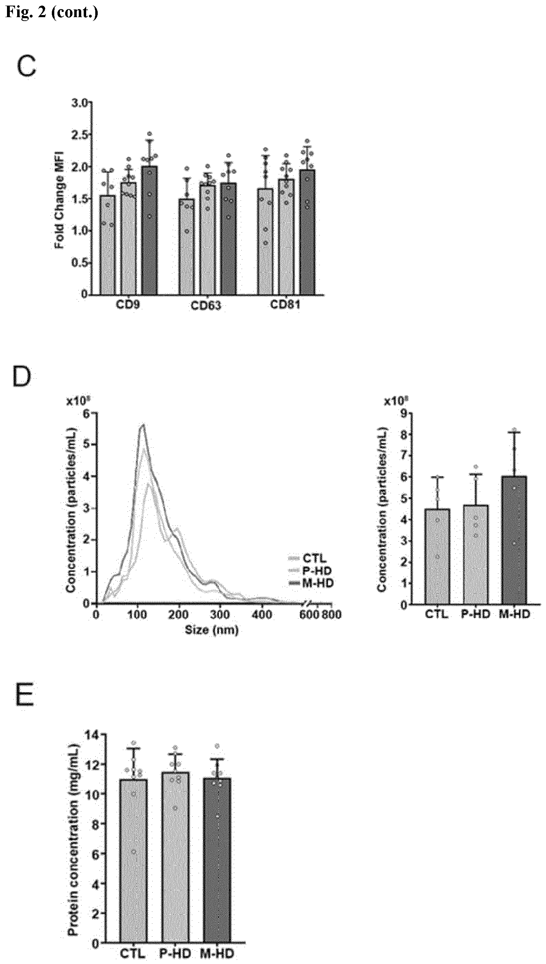

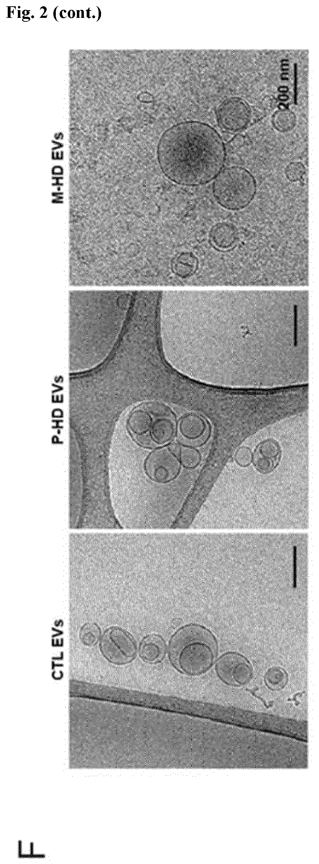

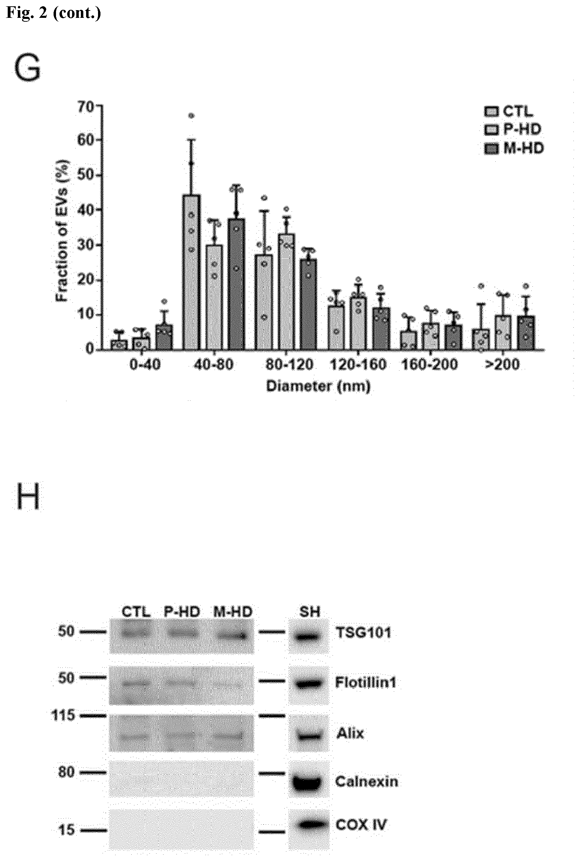

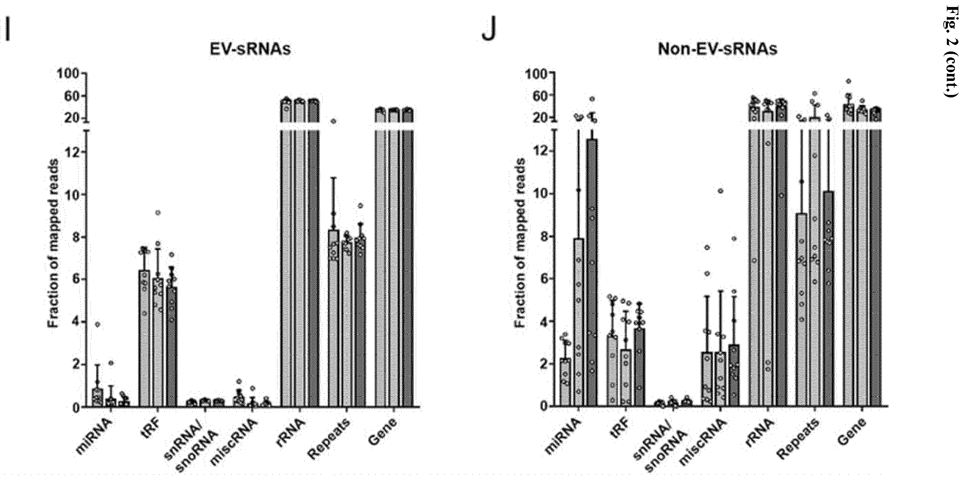

**Figure 2****. Characterization of EV fraction and Non-EV fraction isolated from P-HD, M-HD, and CTL plasma. (A)** Schematic representation of the workflow followed in both plasma subfractions. **(B)** Representative profile of CTL and HD plasma SEC fractions by protein determination and EVs fractions eluted in low protein fractions were detected using EV-markers CD9, CD63 and CD81 by flow cytometry analysis. **(C)** Fold Change of MFI values for CD9, CD63 and CD81 in EVs pooled fractions (n=8-10 per group). **(D)** NTA size distribution profiles of EVs fractions (left) and quantification of EV particle concentration (right) (n=5 per group). **(E)** Protein content of Non-EVs fractions (n=8-10 per group). **(F)** Images of cryo-EM of P-HD, M-HD, and CTL EVs. Bar=200 nm. **(G)** Size distribution of EVs diameter by cryo-EM (n=5 per group). **(H)** Western Blot analysis of EVs fractions for the presence of EV markers (Flotillin1, TSG101 and Alix) and negative EV-markers (COX IV and Calnexin). SH-SY5Y cell lysate (SH) was used as positive control (n=3 per group). sRNA profiles analyzed by SeqCluster tool showing the fraction of reads that align to small RNA types per plasma group **(I)** in EVs fractions and **(J)** Non-EVs fractions (n=10 per group). All data are represented as Mean ± SD.

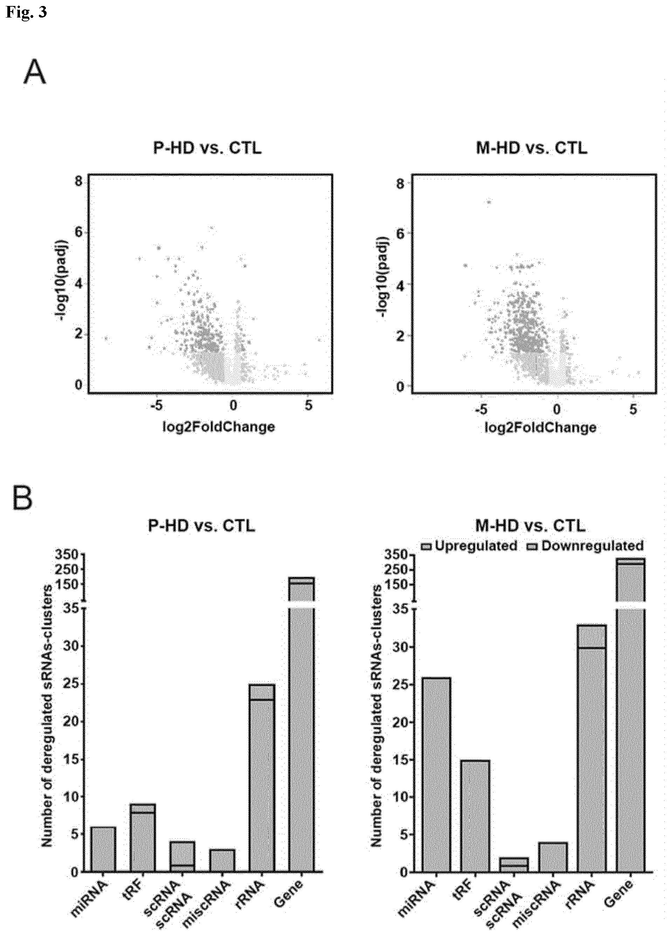

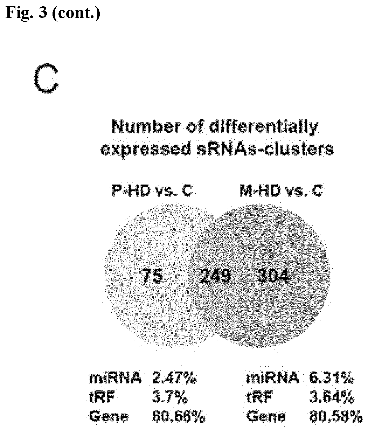

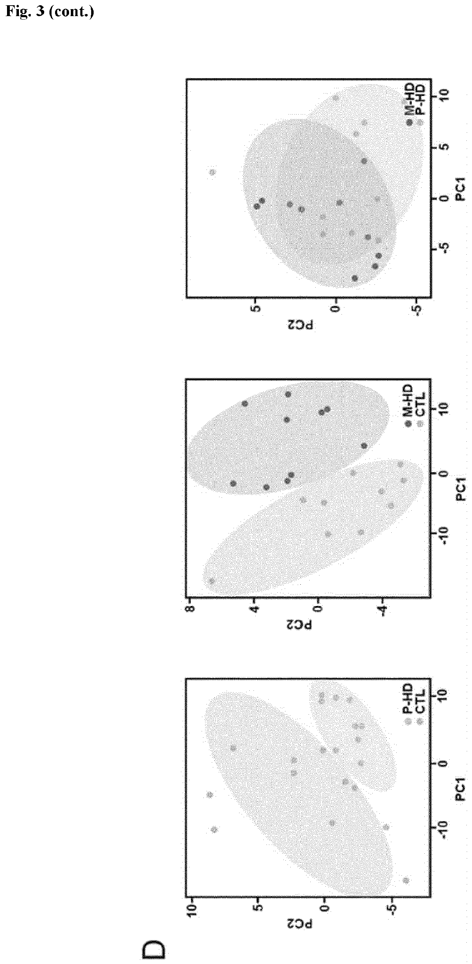

**Figure 3****. sRNAs from HD patients are deregulated in EVs fractions in comparison to CTL samples, using SeqCluster tool. (A)** Volcano plots showing differentially expressed sRNAs-clusters in P-HD-EVs versus CTL-EVs fractions and M-HD-EVs versus CTL-EVs fractions. Orange dots represent significantly deregulated genes (|log2FoldChange|>0.58, adjusted P<0.05). **(B)** Total number of sRNAs-clusters differentially expressed between P-HD-EVs versus CTL-EVs fractions and M-HD-EVs versus CTL-EVs fractions. **(C)** Venn Diagram of differentially expressed sRNAs-clusters between P-HD vs CTL and M-HD vs CTL, showing the number of overlapped dysregulated sRNAs-clusters between both comparisons. **(D)** PCA plots constructed with top DE sRNAs (with PLS-DA based VIP > 0.8) that most contribute to discriminate between disease conditions and healthy individuals. n=10 per group, adjusted P<0.05.

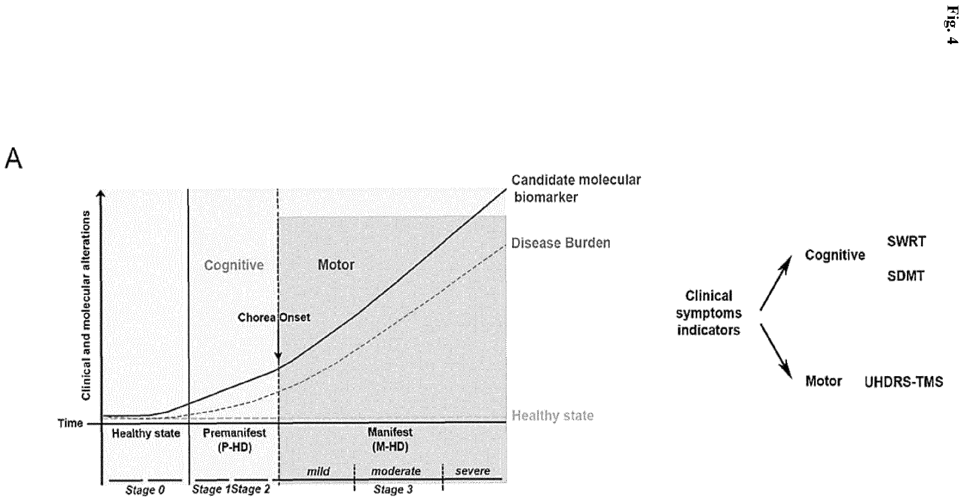

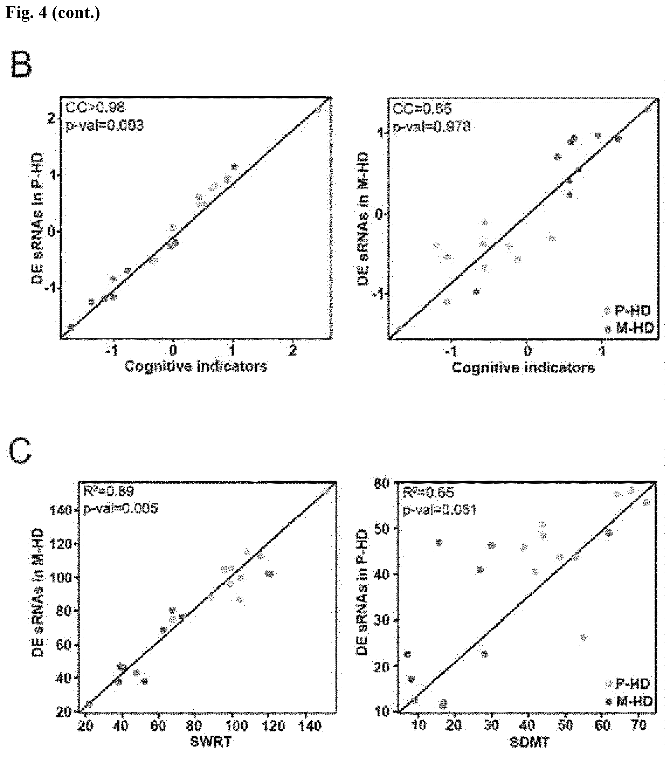

**Figure 4****. sRNAs deregulated in P-HD correlate with cognitive symptomatology. (A)** Cognitive and motor clinical parameters used for correlation analysis and representation of currently proposed rating scales of HD. The ideal candidate molecular biomarker would be measurable prior to the appearance of P-HD and M-HD symptoms and would correlate with the disease course. **(B)** Canonical correlation analysis depicting the relation in HD between top DE sRNAs in P-HD patients (left panel) and top DE sRNAs in M-HD (right panel) and cognitive clinical parameters. **(C)** PLSR analysis depicting the relation between SWRT (R²=0.97; p-val<0.001) and SDMT (R²=0.54; p-val=0.094) and the top DE sRNAs in P-HD patients.

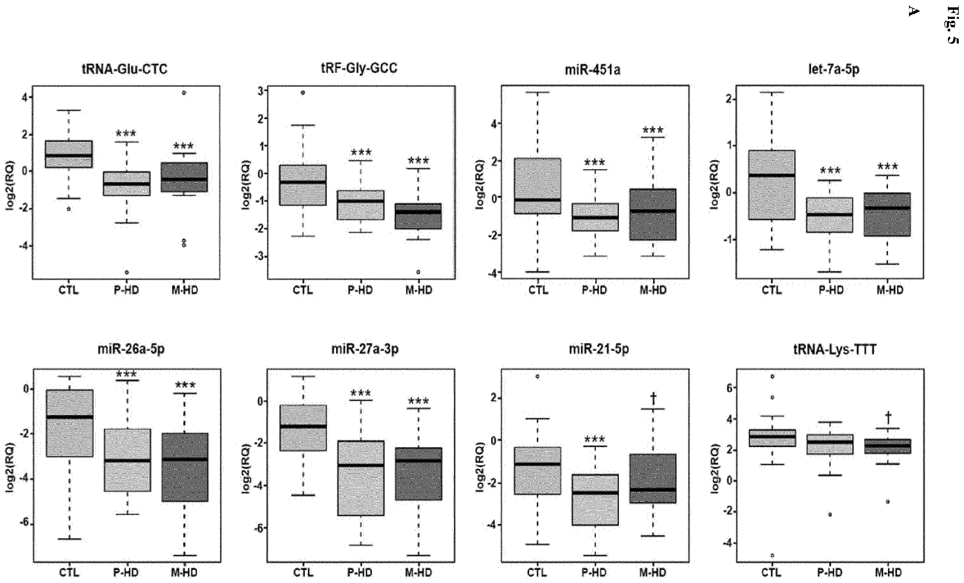

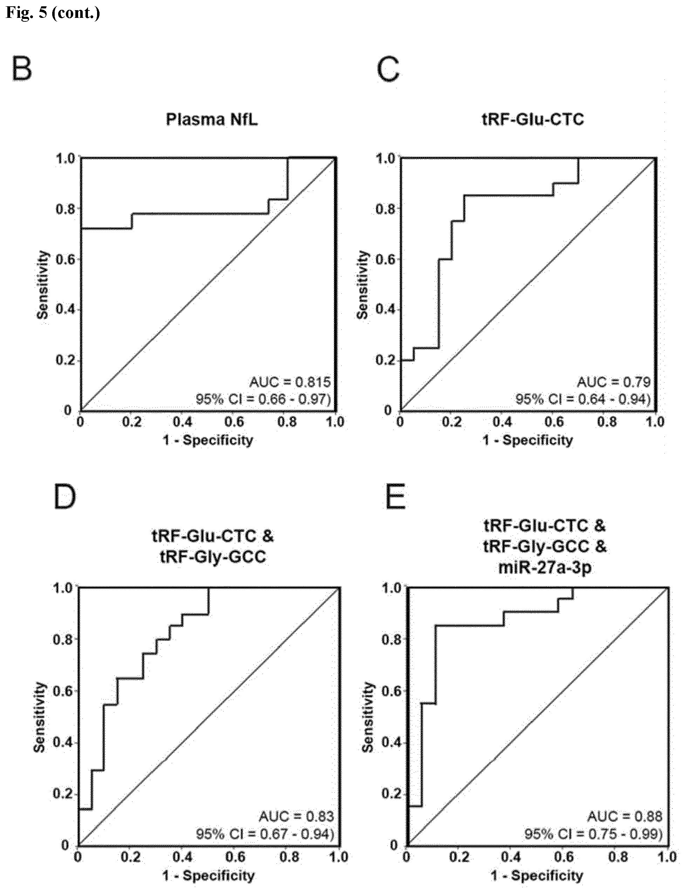

**Figure 5****. Validation and diagnostic potential analysis of selected sRNAs at premanifest stages. (A)** Boxplots representing the relative expression of sRNAs validated with RT-qPCR in technical and biological set of samples. Significant differences between groups are presented with *** (adj P <0.001) and nominally significant differences are presented with † (p-val < 0.05). **(B)** ROC curves analysis of the sensitivity and specificity of plasma NfL between P-HD and CTL (p-val=0.002). **(C)** Representative ROC curve analysis of the sensitivity and specificity of an individual validated sRNA: tRNA^{Glu/CTC} between P-HD and CTL (p-val=0.002). **(D)** ROC curves analysis of the sensitivity and specificity of a novel 2-sRNAs-biosignature: ensemble of tRNA^{Glu/CTC} and tRNA^{Gly/GCC} between P-HD and CTL (p-val=0.0001). **(E)** ROC curves analysis of the sensitivity and specificity of a novel 3-sRNAs-biosignature: the ensemble of tRNA^{Glu/CTC}, tRNA^{Gly/GCC} and miR-27a-3p between P-HD and CTL (p-val=0.0001). n=20 per group.

### Detailed description of the invention

The present invention is illustrated by means of the Examples set below, without the intention of limiting its scope of protection.

### Example 1. Material and methods

### Example 1.1. Clinical samples

All plasma samples from this study were obtained at the Movement Disorders Unit at Hospital de la Santa Creu i Sant Pau (Barcelona, Spain).

We recruited forty-one HD patients and twenty-two healthy non-mutation carrier control participants, age- and gender-matched. Individuals were confirmed gene mutation carriers (CAG repeat length > 40), and disease-specific indicators including motor, cognitive, and neuropsychiatric status, were recorded.

The disease burden score (DBS) was used as an index of pathological burden due to lifetime exposure to mutant huntingtin and calculated as age (in years) × (CAG - 35.5). HD patients were grouped into premanifest (P-HD, n=21) and manifest (M-HD, n=20) stages based on their Unified Huntington's Disease Rating Scale (UHDRS). The diagnostic confidence level, the total functional capacity (TFC), motor symptoms (TMS), and global cognitive status (Cogscores) were considered. Participants were classified as M-HD patients if UHDRS-TMS > 5 and a diagnostic confidence level=4, indicating that motor abnormalities were unequivocally caused by HD with ≥99 % of confidence. The following cognitive indicators, known to be sensitive to HD progression, were included: Symbol Digit Modality Test (SDMT) and the Stroop-word task (SWRT). Composite UHDRS (cUHDRS) scores were also considered, given its improved implementation as a measure of disease progression in clinical trials.

All participants provided written informed consent previously approved by the appropriate ethics committee and was therefore performed in accordance with the ethical standards laid down in the 1964 Declaration of Helsinki and its later amendments.

### Example 1.2. Sample collection and processing

Blood samples were collected in tubes with 0.109 M 3.2% trisodium citrate (Vacutainer, Becton Dickinson) via antecubital vein puncture in the morning, and processed within 30 min after withdrawal. Tubes were centrifuged at 500 g for 10 min at RT and the supernatant was pipetted off from cell debris. The supernatant was centrifuged again at 2,500 g for 15 min at RT in order to eliminate platelets and one more time at 13,000 g for 15 min at RT to eliminate bigger particles and smaller aggregates. Platelet-free plasma was collected and stored directly at -80°C in one mL aliquots.

### Example 1.3. Plasma NfL

Plasma NfL levels were measured with the Simoa human NF-light Advantage kit using the Single Molecule Array (Simoa) technology (Simoa, Quanterix) in a SR-X Biomarker detection system by following the manufacturer's instructions.

### Example 1.4. Plasma fractionation and EVs isolation

For plasma fractionation and EVs isolation, we used size-exclusion chromatography (SEC) followed by ultrafiltration (UF). Briefly, Sepharose CL-2B (Sigma Aldrich, MO, USA) was stacked in Puriflash Dry-Load Columns (Interchim) of 20 mL. On top of each column, two mL of plasma were loaded, and fractions of 500 µL were collected manually in individual tubes (a total of 35 fractions) using filtered 1X PBS as elution buffer. Protein content for each fraction was measured by Micro BCA Protein Assay Kit (Thermo Fisher Scientific) and Bio-Rad Protein Assay Dye Reagent (BioRad). SEC-fractions were further characterized using classical EVs-associated markers CD9, CD63, and CD81 by bead-based flow cytometry assay from BD LSRFortessa (BD Biosciences). Median fluorescence intensity (MFI) values were obtained using Diva Software, and the four fractions with the highest fold change MFI compared to the isotype control were considered EVs-enriched fractions and were pooled.

Purified EVs were further concentrated by ultrafiltration using Amicon Ultra 100 KDa Centrifugal Filters (Merck Millipore) according to the manufacturer's instructions, obtaining the concentrated EVs (cEV fraction) and Filtrate fractions. Pooled fractions were kept at - 80°C until later use.

For the determination of sRNA diversity in the different plasma subfractions, four SEC fractions depleted of EVs with low protein concentration (LowP fraction) and four SEC fractions depleted of EVs with high protein content (HighP fractions) were also pooled.

For the analysis of expression patterns of plasma sRNAs in HD, eight EV-depleted SEC fractions (identified as Non-EVs fraction) were pooled with the aim to investigate the extravesicular fraction in the patients' and healthy controls' groups.

### Example 1.5. Nanoparticle tracking analysis

The particle size distribution and concentration measurements in cEVs fractions were evaluated by nanoparticle tracking analysis (NTA) using a NanoSight NS300 instrument (Malvern Panalyitical) and following the manufacturer's instructions. Each sample was diluted in sterile and filtered 1X PBS to the working range of the system (106-109 particles/mL). Data acquisition and processing were performed using the Nanosight NS300 software (version 3.4) recording three 30-second videos with 25 frames per second. Camera type was sCMOS with Laser Type Blue488 and Camera Level 12. Video analysis settings were set to a detection threshold of 5.

### Example 1.6. Western Blotting

Assessment of specific EV markers and Non-EVs markers was performed by Western blotting. Protein concentration was measured by Micro BCA Protein Assay Kit (Thermo Fisher Scientific) in the cEVs and Filtrate fractions, samples with low input of protein. Protein concentration in LowP fractions, HighP fractions, and cell lysate samples was determined using Bio-Rad Protein Assay Dye Reagent (BioRad). Equal protein amount of each sample (20 µg) was mixed with reducing 5X Pierce Lane Marker Reducing Sample Buffer (Thermo Fisher Scientific) and boiled for 5 min at 95°C. Due to their not-existing protein content, for Filtrate fractions, the same volume as the one corresponding to 20 ug of the cEVs fractions (from original sample), was loaded. Samples were resolved in NuPAGE 4-12% Bis-Tris polyacrylamide gels (Thermo Fisher Scientific) using MOPS SDS as running buffer (Thermo Fisher Scientific). Gels were transferred to nitrocellulose membranes with the iBlot2 system (Thermo Fisher Scientific). In order to visualize the depletion of Albumin and IgGs in cEVs fractions, Ponceau S staining (Sigma Aldrich) was applied. Membranes were blocked with 5% non-fat dry milk (sc-2325, Santa Cruz Biotech) diluted in 1X Tris-buffered saline with 0.1% Tween20 (TBS-T) for 1 h at RT. Blots were incubated overnight at 4 °C with the following primary antibodies: rabbit anti-CD9 (13174, Cell Signalling Technology), rabbit anti-Alix (12422-1-AP, Proteintech), mouse anti-Flotillin1 (610820, BD Biosciences), rabbit anti-TSG101 (ab30871, Abcam), rabbit anti-Syntenin (ab133267, Abcam), rabbit anti-Calnexin (ab22595, Abcam) or mouse anti-OxPhos Complex IV subunit IV (A-21347, Thermo Fisher Scientific) diluted 1:1000 in TBS-T with 1% Bovine Serum Albumin (BSA, Sigma). Membranes were then washed three times in TBST-T and incubated for 1 h at RT with goat anti-rabbit or goat anti-mouse HRP conjugated secondary antibodies (31460 and 31430, respectively, Thermo Fisher Scientific) diluted 1:10000 in TBS-T. Blots were washed again three times in TBS-T and detected with SuperSignal West Pico Plus or SuperSignal West Femto Maximum Sensitivity chemiluminescent substrates (Thermo Fisher Scientific). Images were acquired in the imaging system Chemidoc (BioRad).

### Example 1.7. Cryo-transmission electron microscopy

Plasma subfractions were imaged by cryogenic transmission electron microscopy (cryo-TEM). Vitrified specimens were prepared by placing 3 µL of a sample on a Quantifoil 1.2/1.3 TEM grid, blotted to a thin film, and plunged into liquid ethane-nitrogen in the Leica EM-CPC cryo-work station. The grids were transferred to a 626 Gatan cryo-holder and maintained at -179 °C. The grids were analyzed with a Jeol JEM 2011 transmission electron microscope operating at an accelerating voltage of 200 kV. Images were recorded on a Gatan Ultrascan 2000 cooled charge-coupled device (CCD) camera with the Digital Micrograph software package (Gatan). Size distribution of EVs was quantified by measuring the diameter of around 200 vesicles in each sample using ImageJ.

### Example 1.8. RNA extraction and small RNA sequencing

sRNA was isolated using the miRNeasy Serum/Plasma Advanced Kit (Qiagen) according to the manufacturer's instructions. All samples were adjusted to an input final volume of 200 µL. RNA samples were eluted in 20 µL of RNAse-free water. Prior to sequencing, RNA was precipitated using glycogen (20 mg/ mL, Roche), sodium acetate 3M pH 4.8 (Sigma-Aldrich) and 100% ethanol. After 24 h at -20°C, the mixtures were centrifuged at 14,000 rpm for 30 min at 4°C. Carefully, the supernatant was removed, and the pellet was then washed with 75% ethanol followed by centrifugation at 12,000 rpm for 5 min at 4°C. Finally, the supernatant was discarded, and the RNA pellet was dried at RT and dissolved at 65°C for 3 min in 6 µL of RNAse-free water and stored keeping the cold chain at -80°C.

The RNA samples were analyzed for RNA quality and level of degradation using the Bioanalyzer 2100 (Agilent). sRNA libraries were generated using the NEBNext Small RNA Library Preparation Set for Illumina (New England Biolabs) following the manufacturer's instructions. Indexed libraries were equimolarly pooled, and the selected band of interest (145-160 bp) was purified. Single-end sequencing of libraries was then carried out on the Illumina HiSeq 2000 with read lengths of 50 nt, generating at least 8 million reads per sample.

### Example 1.9. Bioinformatic and biostatistical analysis

The quality of the sequenced fastq files was checked using the FastQC software (http://www.bioinformatics.babraham.ac.uk/projects/fastqc/). Adapter trimming was performed with Cutadapt and the trimmed reads were aligned to the human genome (version Ensemble hg 19) using STAR aligner (version 2.7.9a). The obtained sequences were annotated and quantified using SeqCluster and ExceRpt bioinformatic tools. Prior to further analyses, only sRNAs identified with a minimum of ten counts in at least half of the samples of the tested conditions were considered. Differential expression analyses were performed through negative binomial generalized models implemented in the Bioconductor package DESeq2 (Love et al., 2014) from R statistical software. Benjamini-Hochberg correction of 0.05% for multiple testing was applied to control the False Discovery Rate.

Partial least squares discriminant analysis (PLS-DA), a multivariate supervised method, based on orthogonal transformations of the initially quantified sRNAs into few uncorrelated components in a way that maximizes the separation of each disease condition, was performed as previously described. Three PLS-DA models were applied for each of the three sRNA biotypes considered (miRNAs, tRFs and gene fragments). To elude over-parameterized models with rather poor discriminant properties, we obtained in a first stage of the process, the most important discriminant clusters from the three PLS-DA models. And, in a second stage, we conducted another PLS-DA analysis including the important clusters associated independently to the three different models found in the first stage. Variable importance for the projection (VIP) criterion was used to identify which sRNAs contribute most to the classification performance, and sRNAs with VIP > 0.8 were considered for further downstream analysis. Principal Component Analysis (PCA) was applied as a non-supervised method to evaluate the separation between disease conditions using the top contributing sRNAs based on PLS-DA analysis. For both PLSA-DA and PCA analyses count matrices normalized and transformed to variance-stabilized expression levels were used.

Pearson's coefficient (r) was used to screen out the correlation between individual selected top DE sRNAs (DE sRNAs with p-adj < 0.05 and PLS-DA based VIP > 0.8) and each clinical parameter. The selection criteria included sRNAs with r > 0.7 and p-value < 0.001.

Canonical-correlation analysis (CCA) was applied in order to find linear combinations of the top DE sRNA and sets of clinical variables which have maximum correlation with each other. Partial least squares regression (PLSR) model was applied to decipher which clinical parameter was better explained by the selected top DE sRNAs. Permutation tests were applied for statistical significance purposes.

### Example 1.10. qRT-PCR validation

Half of the volume of EVs and Non-EVs pooled fractions (1 mL) was used for RNA isolation as described in the section "RNA extraction and small RNA sequencing" with minor modifications. Prior to RNA sample lysis using RPL buffer, synthetic RNA spike-ins (UniSp2, UniSp4, UniSpS RNA Spike-in mix; Qiagen) were added and used as controls for RNA isolation, in accordance with the kit protocol. Two µL of isolated RNA were used for reverse transcription (RT) and cDNA synthesis using the miRCURY LNA RT Kit (Qiagen) according to the manufacturer's instructions adding the RNA spike-in UniSp6 as RT control. The obtained cDNA was further diluted 1:10 for qPCR and, 3 µL were used as template for the qPCR in a 10 µL final volume reaction using the miRCURY LNA SYBR Green PCR Kit (Qiagen) in a StepOnePlus Real-Time PCR System (Applied Biosystems). For each determination, reactions were performed in triplicate.

Due to the absence of universal endogenous sRNA normalizers in EVs, miR-100 was selected as the housekeeping gene since it was the most stable sRNA with the least variability among all the sequenced samples. For the analysis of relative quantification (RQ) of qRT-PCR data, linear mixed-effects models (LMM) were applied that accounted for the different sources of variation derived from the experimental design.

The diagnostic accuracy of validated sRNAs was explored by receiver operating characteristic (ROC) curves. For the analysis of sRNA combinations/biosignatures, logistic binary regression was applied, and the obtained estimated probabilities were used to build ROC curves. Area under the ROC curve (AUC) and its 95% confidence interval were used as the diagnostic accuracy metric.

### Example 2. Results

### Example 2.1. Different plasma subfractions show specific sRNA profiles

Aiming to understand exRNA diversity in plasma samples, we first profiled sRNA diversity in different plasma subfractions. We applied the already well-established EV-isolation protocol based on Size Exclusion Chromatography (SEC) followed by Ultrafiltration (UF) in five plasma samples from healthy volunteers, obtaining four well-differentiated plasma subfractions: cEVs, concentrated EVs-enriched fraction after UF of tetraspanin-enriched SEC fractions; Filtrate, elute obtained after UF of SEC-EVs; LowP, pooled tetraspanin-negative SEC fractions with low protein concentration; and HighP, non-EV fractions with high protein concentration as shown by Bradford protein assay (**Fig. 1A**). SEC-eluted fractions enriched in EVs were determined by flow cytometry analyzing the presence of tetraspanins CD9, CD63 and CD81 (**Fig. 1B**). The four fractions showing positivity for tetraspanins were pooled (EVs-enriched fractions) and subsequently concentrated by UF (cEVs). cEVs were further characterized in compliance with the MISEV guidelines. EV concentration yielded 8.33 × 10⁹ ± 2.65 × 10⁸ particles/mL with a mean diameter size of 146.0 ± 2.8 nm in cEVs fractions as determined by NTA (**Fig. 1C**). Western Blot was used to confirm the presence of additional EV markers (Alix, TSG101, Syntenin, Flotillin1, and CD9) only in cEVs fractions and the absence of them in Filtrate, LowP, and HighP fractions. Negative expression for cellular markers of endoplasmic reticulum and mitochondria (Calnexin and COX IV, respectively) verified the optimal purification of EVs. Neuronal SH-SY5Y cell lysate was used as a positive control for the different markers (**Fig. 1D**). The depletion of Albumin and IgGs in cEVs fractions was confirmed by Ponceau red staining of the membrane. Subfractions were also evaluated using cryo-TEM confirming the presence of EVs with an expected range of size and morphology features in cEV fractions, and absence of EVs in Filtrate, LowP and HighP fractions (**Fig. 1E**).

To uncover the sRNA diversity in the different plasma subfractions, we performed sRNA-sequencing in cEVs, Filtrate, LowP, and HighP, obtained from five healthy volunteers. For sRNA annotation and quantification, SeqCluster and ExceRpt bioinformatic pipelines were used. ExceRpt is a reference tool used to characterize all types of circulating sRNAs. During the pre-processing step, it filters out highly abundant sRNAs mapping onto ribosomal RNAs (rRNAs); however, recent data suggest that these fragments of rRNAs are bioactive molecules (doi:10.1038/s41556-021-00652-7). In addition, ExceRpt only considers the type of isoacceptor when identifying tRNA-derived fragments (tRFs), leading to an oversimplification of the tRFs landscape that has been revealed as highly complex. It has been described that diverse types of tRFs mapping onto different parts of the mature tRNA are differently expressed under physiological and pathological conditions, which makes them appealing biomarkers (doi: 10.1080/15476286.2020.1729584). For these reasons, in order to have a more in-depth and complementary vision of the plasma exRNA transcriptome, we used both tools. Specifically, SeqCluster tool detects units of sRNAs (clusters) using a heuristic iterative algorithm to deal with multi-mapped events. Sequences are consistently and non-redundantly grouped if they map onto the same genomic site defining "clusters". Clusters are then classified according to the type of precursor where sRNAs map, including rRNA-clusters and tRF-clusters.

We detected heterogeneous proportions regarding sRNAs biotypes between the different plasma fractions using SeqCluster (**Fig. 1F**) and ExceRpt tools. We noted that cEVs fractions contained a higher percentage of reads mapping onto miRNAs than Filtrate, LowP, and HighP fractions. However, the highest proportion of reads mapping onto gene fragments was found in HighP fractions (**Fig. 1F**). SeqCluster analysis showed that other sRNAs biotypes, such as tRFs, were homogenously distributed in the four fractions, while rRNAs and repeats fragments were found in less proportion in HighP fractions than in other fractions. In addition, tRF fragments were classified based on their mapped positions (3'-half, 3'-tRF, i-tRF, 5'-tRF, and 5'-half) and similar distributions were observed in the different subfractions (**Fig. 1**G). Focusing on the total number of different sRNA clusters identified in each subfraction, we observed that, in accordance with the relative abundance, cEVs fractions contained an increased number of miRNA clusters. This result indicates that although miRNAs were a minor proportion of the total sRNAs, they were concentrated in cEVs compared with other fractions. Despite the higher abundance of gene fragments in HighP fractions, a similar diversity was detected in the different plasma subfractions. Similar results of the diversity of gene fragments were found using the ExceRpt tool. Moreover, using ExceRpt, we were able to subclassify the reads mapping onto gene fragments. Hence, the increased proportion of gene fragments in HighP fraction was mainly due to protein coding and retained intron fragments. Through hierarchical clustering analysis, we likewise assessed the differential sRNA transcriptome in the different fractions: heatmap analysis based on miRNAs expression consistently showed a clear grouping of cEVs fractions: 4 out of 5, using SeqCluster and 5 out of 5, using ExceRpt; similarly, all HighP fractions were clustered when gene fragments clusters expression was considered, utilizing SeqCluster and ExceRpt. Of notice, differential expression analysis identified 26 upregulated and 0 downregulated miRNA-clusters, and 61 upregulated and 139 downregulated gene fragments-clusters in cEVs in comparison to HighP fractions (using SeqCluster, **Fig. 1H**). Similar results were found using ExceRpt tool. These findings suggest that both, cEVs and HighP fractions, shows distinct patterns of sRNA distribution and may provide specific and complementary information about changes and expression patterns of sRNAs, which would provide new insights for biomarkers discovery.

### Example 2.2. No morphological and size differences were found between plasma CTL- and HD-EVs

Once the general profiling of sRNA diversity in plasma subfractions was established, we next sought to analyze the expression pattern of plasma exRNAs in HD. For this purpose, three different groups of samples were considered: premanifest HD patients (P-HD), manifest HD patients (M-HD), and healthy control volunteers (CTL). We then selected two well-differentiated plasma fractions, the EV fraction, enriched in cEVs and the Non-EVs fraction, depleted of EVs and showing a high protein concentration (**Fig. 2A**). According to our previous analysis, these fractions might be informative and complementary from a biomarker perspective. EVs were further characterized based on MISEV guidelines and compared between the three groups. Regarding flow cytometry profiling for tetraspanins expression, we did not observe differences between groups (**Fig. 2B****,** **2D**). Likewise, no differences were observed in EVs concentration nor size between groups as measured by NTA (**Fig. 2D**). In Non-EVs fractions, the measurement of total protein concentration in the different groups rendered similar results (**Fig. 2E**). Using cryo-EM, we confirmed the size and integrity of EVs (**Fig. 2F**), and we verified that similar vesicular size distribution was observed in M-HD, P-HD and in CTL samples (**Fig. 2G**). Western Blot was used to confirm the presence of TSG101, Flotillin1, and Alix, and the absence of Calnexin and COX IV in EVs (**Fig. 2H**). Moreover, Ponceau red staining in the two fractions illustrated the depletion of highly abundant Albumin and IgGs in EVs fractions.

### Example 2.3. Heterogeneous diversity of extracellular sRNAs in HD and CTL samples

To uncover the sRNA diversity in the two plasma fractions selected between HD and control samples, we characterized the sRNA profiles by deep sequencing and applied diverse supervised and unsupervised statistical methods to ascertain sRNA expression differences (**Fig. 2A**). Similar to what we observed in the proof of concept, the majority of reads mapped onto rRNA (51%) followed by gene fragments (34%), and a comparable total number of different sRNA clusters were identified in the three groups. However, we observed heterogeneous proportions of some sRNAs biotypes in the two plasma fractions, including miRNAs and tRFs (**Fig. 2I, 2J**). It is worth mentioning that sequencing analyses revealed low levels of miRNAs which accounted for less than 2% of the EVs-sRNA content (**Fig. 2I**), although they have received major attention in biomarker discovery. Noticeably, Non-EVs fractions showed greater intragroup variability (**Fig. 2J**). In contrast, in the EVs fractions, probably due to the conferred vesicular protection, the proportions of sRNAs are more homogeneous between individuals from the same group. All the analyses were also performed using the ExceRpt tool, obtaining similar results.

Taking into consideration our results from the plasma subfractions sRNA profiling, we expected to explore and define HD biomarkers in both, EVs- and Non-EVs plasma fractions. Nevertheless, due to the great variability observed in the analysis of Non-EV fraction, we opted to proceed with the downstream analyses only in the vesicular compartment (EVs fraction).

### Example 2.4. exRNAs-EVs are deregulated in HD and correlated with early symptomatology

Focusing on EVs fraction, through differential expression analysis, our results highlighted a significant downregulation of most of the sRNAs in HD compared to CTL samples, with many changes commonly occurring at premanifest stages (**Fig. 3A, 3B**). Most of these deregulated sRNAs were found to be annotated as miRNAs, tRFs, and gene fragments (**Fig**. **3C**). We applied PLS-DA model to highlight specific expression patterns of sRNAs discriminating groups. miRNAs, tRFs, and gene fragments that contributed more to the PLS-DA discrimination (top DE sRNAs with PLS-DA based VIP > 0.8) were selected for unsupervised principal component analysis (PCA). The first two components of PC analysis showed that HD patients, both premanifest and manifest, clearly separated from healthy controls (**Fig. 3D**). All the analyses were also performed using the ExceRpt tool, obtaining similar results.

To gain insight into the association of top DE sRNAs (miRNAs, tRFs, and gene fragments) and the disease, we explored the correlation between their expression levels and patients' clinical features considered during the HD course. First, using Pearson's coefficient and applying these cut-offs (DE miRNAs, tRFs, and gene fragments showing a p-adj < 0.05 and PLS-DA based VIP > 0.8), we identified three different sets of top DE sRNAs that individually correlated with any of the clinical features: 12 DE sRNAs in P-HD, 23 DE sRNAs in P-HD and in M-HD, and 57 sRNAs DE in M-HD. We then applied canonical correlation analysis (CCA) grouping SWRT and SDMT tests as a "cognitive scale" (**Fig. 4A**), and the different sets of top DE sRNA were studied for correlation. These analyses showed that the set of top DE sRNAs in P-HD was significantly related to the cognitive status of HD patients (**Fig 4B**, left panel; canonical correlation (CC) > 0.98; p-val=0.003). Similar analyses considering the set of top DE sRNAs in M-HD (**Fig 4B**, right panel; CC=0.65; p-val=0.978) or the top DE sRNAs in both P-HD and M-HD patients did not show a good correlation (CC=0.63; p-val=0.786). These results indicate that sRNAs specifically DE in the premanifest stage are good enough to highlight the early cognitive symptoms. To define in detail this correlation, we performed a PLSR analysis considering SWRT and SDMT individually. Noticeably, we observed that the expression pattern of the top DE sRNAs in P-HD patients was related to the performance of the SWRT test by HD patients (coefficient of determination (R²) = 0.89; p-val = 0.005) but not to SDMT scores (R²=0.65; p-val=0.061) (**Fig. 4C**). To assess to what extent the correlation observed between SWRT and the set of top DE sRNAs in P-HD is mainly due to the disease and not a consequence of population diversity, we repeated the analyses including CTL samples that were similarly evaluated. In this case, we observed that the correlation coefficients from CCA (CC=0.339 p-val=0.993) and from PLSR (R2=0.104; p-val=0.960) lost significance. Parallel regression analyses with the set of DE sRNAs in both PM-HD and M-HD, and the top DE sRNAs in M-HD patients and UHDRS-TMS as motor assessment also reached a significant correlation when considering only the manifest group of patients (R²=0.662, p-val<0.0001; and R²=0.60, p-val=0.044, respectively). This correlation with motor symptomatology was lost when the premanifest group of patients (UHDRS-TMS score < 5) was included in the analysis. In this case, top DE sRNAs in P-HD did not show any relation to UHDRS motor score.

### Example 2.5. Plasma EV-sRNAs as promising early biomarkers in HD

As potential new biomarkers in HD and aiming to confirm the sRNAs-sequencing results, eight sRNAs candidates (DE sRNAs showing a p-adj < 0.05 in P-HD and in M-HD vs CTL patients, with a base mean sequence coverage > 200 and PLS-DA based VIP > 0.8) were selected for validation by qPCR. The selected sRNAs were tRNA^{Glu/CTC}, tRNA^{Gly/GCC}, miR-451a, let-7a-5p, miR-26a-5p, miR-27a-3p, miR-21-5p, and tRNA^{Lys/TTT}. We validated the relative expression in the same set of samples used in sRNA sequencing and in an independent biological set of samples (total n=20/group). Due to the absence of universal endogenous sRNA normalizers, hsa-miR-100-5p was selected as the reference gene since it was the most stable sRNA with the least variability among all the sequenced samples.

According to qPCR results, tRNA^{Glu/CTC}, tRNA^{Gly/GCC}, miR-451a, let-7a-5p, miR-26a-5p, miR-27a-3p, miR-21-5p, and tRNA^{Lys/TTT} expression were significantly downregulated in P-HD and M-HD in comparison to CTL samples, consistent with the sequencing findings (**Fig. 5A**).

Among all the currently established blood-based biomarkers for HD, neurofilament light chain (NfL) is the most sensitive one, although it is not specific to HD, but refers to neuronal damage. In HD, NfL shows the highest specificity and sensitivity when defining M-HD patients, but this performance in our group of P-HD patients decreases to an AUC = 0.815; CI= 0.66 - 0.97 (**Fig. 5B**).

In an attempt to define better and more sensitive biomarkers for the early stages of the disease, we compared the biomarker prediction outcome of NfL and our sRNA biosignature in the P-HD set of samples used for qPCR analysis. Our results showed that validated sRNAs could be considered promising biomarkers for P-HD stage as they individually show a similar diagnostic potential compared to NfL (**Fig. 5C**). Of note, our data indicated that the combination of two or three validated sRNAs showed similar specificity and sensitivity discriminating between P-HD patients and healthy individuals, with a higher AUC (**Fig. 5D**, **5E**). For instance, the performance of an ensemble of 2-sRNAs-biosignature including tRNA^{Glu/CTC} and tRNA^{Gly/GCC} presented a higher AUC = 0.83 (CI = 0.67-0.94, **Fig. 5D**); and an ensemble of 3-sRNAs-biosignature being tRNA^{Glu/CTC}, tRNA^{Gly/GCC} and miR-27a-3p, an AUC = 0.88 (CI = 0.75 - 0.99), **Fig. 5E**).

Likewise, using these set of patients and considering the use of plasma NfL in combination with our validated sRNAs, we estimated a higher diagnosis potential. Specifically, the combined biosignature of tRNA^{Gly/GCC}, miR-27a-3p and miR-451a, together with plasma NfL showed an AUC=0.904 (CI = 0.8 - 1). Although the amelioration of the diagnostic accuracy was not significant, we obtained a CI more precise.

## Claims

1. *In vitro* method for the diagnosis or prognosis of Huntington's disease which comprises assessing the level of expression of a tRNA fragment which comprises the 5'end of tRNA^{Glu/CTC} (tRF-5^{Glu/CTC}) or 5'end of tRNA^{Gly/GCC} (tRF-5^{Gly/GCC}) in a biological sample obtained from the subject, wherein the identification of a deviation in the expression level with respect to a pre-established threshold value determined in healthy subjects is an indication that the subject may be suffering from Huntington's disease or has a poor prognosis.

2. *In vitro* method for the diagnosis or prognosis of Huntington's disease, according to claim 1, wherein the tRF-5^{Glu/CTC} sequence is selected from Table 13.

3. *In vitro* method for the diagnosis or prognosis of Huntington's disease, according to any of the previous claims, which comprises assessing the level of expression of a tRF-5^{Glu/CTC} derived from the following tRNAs: tRNA^{Glu-CTC-1-1}, tRNA^{Glu-CTC-2-1}, tRNA^{Glu-CTC-1-5}, tRNA^{Glu-CTC-1-4}, tRNA^{Glu-CTC-1-3}, tRNA^{GluCTC-1-6}, tRNA^{Glu-CTC-1-2} or tRNA^{Glu-CTC-1-7}.

4. *In vitro* method for the diagnosis or prognosis of Huntington's disease, according to claim 1, wherein the tRF-5^{Gly/GCC} is selected from Table 14.

5. *In vitro* method for the diagnosis or prognosis of Huntington's disease, according to any of the claims 1 or 4, which comprises assessing the level of expression of a tRF-5^{Gly/GCC} derived from the following tRNAs: tRNA5^{Gly-GCC-5-1}, tRNA5^{Gly-GCC-3-1}, tRNA5^{Gly-GCC-2-1}, tRNA5^{Gly-GCC-2-5}, tRNA5^{Gly-GCC-2-4}, tRNA5^{Gly-GCC-2-6}, tRNA5^{Gly-GCC-2-2} or tRNA5^{Gly-GCC-2-3}.

6. *In vitro* method for the diagnosis or prognosis of Huntington's disease, according to any of the previous claims, which further comprises assessing the level of expression of miR-451a, miR-21-5p, miR-27a-3p, let-7a-5p, miR-26a-5p, a tRNA fragment comprising the 5'end of tRNA^{Lys/TTT} and/or Neurofilament light chain (NfL), or combinations thereof.

7. *In vitro* method for the diagnosis or prognosis of Huntington's disease, according to any of the previous claims, which comprises assessing the level of expression of the combination of biomarkers of Table 12.

8. *In vitro* method, according to any of the previous claims, which comprises a) measuring the expression level of the combinations of biomarkers of claims 6 or 7, b) processing the expression level values in order to obtain a risk score, and c) wherein if the risk score obtained has a variation or deviation as compared with a reference value, this is indicative that the subject is suffering from Huntington's or has a poor prognosis.

9. *In vitro* use of a tRF-5^{Glu/CTC} or tRF-5^{Gly/GCC}, or of a kit comprising reagents for the detection of tRF-5^{Glu/CTC} or tRF-5^{Gly/GCC}, for the diagnosis or prognosis of Huntington's disease.

10. *In vitro* use, according to claim 9, wherein the tRF-5^{Glu/CTC} is selected from Table 13.

11. *In vitro* use, according to claims 9 or 10, of a tRF-5^{Glu/CTC} derived from the following tRNAs: tRNA^{Glu-CTC-1-1}, tRNA^{Glu-CTC-2-1}, tRNA^{Glu-CTC-1-5}, tRNA^{Glu-CTC-1-4}, tRNA^{Glu-CTC-1-3}, tRNA^{GluCTC-1-6}, tRNA^{Glu-CTC-1-2} or tRNA^{Glu-CTC-1-7}.

12. *In vitro* use, according to claim 9, wherein the is selected from Table 14.

13. *In vitro* use, according to any of the claims 9 or 12, wherein the tRF-5^{Gly/GCC} is derived from the following tRNAs: tRNA5^{Gly-GCC-5-1}, tRNA5^{Gly-GCC-3-1}, tRNA5^{Gly-GCC-2-1}, tRNA5^{Gly-GCC-2-5}, tRNA5^{Gly-GCC-2-4}, tRNA5^{Gly-GCC-2-6}, tRNA5^{Gly-GCC-2-2} or tRNA5^{Gly-GCC-2-3}.

14. *In vitro* use, according to any of the claims 9 to 13, of a combination of a tRF-5^{Glu/CTC} or tRF-5^{Gly/GCC} with any of the following biomarkers: miR-451a, miR-21-5p, miR-27a-3p, let-7a-5p, miR-26a-5p, a tRNA fragment derived from the 5' end of tRNA^{Lys/TTT} and/or Neurofilament light chain (NfL).

15. *In vitro* use, according to any of the claims 9 to 14, of the combination of biomarkers of Table 12.
